# EUROPEAN PATENT SPECIFICATION

(11) **EP 3 076 985 B1**
(45) Date of publication and mention of the grant of the patent: **24.07.2024**
(21) Application number: 14806649.1
(22) Date of filing: 04.12.2014
(51) Int. Cl.: A61K 35/741, A61K 38/00, A61K 38/55, A61K 39/02, A61K 31/00, A61K 31/11, A61P 3/04

(54) **COMPOSITION COMPRISING PROBIOTICS OVEREXPRESSING CLPB PROTEIN FOR USE IN THE TREATMENT OF OBESITY**
ZUSAMMENSETZUNG ENTHALTEND CLPB PROTEIN ÜBEREXPRIMIERENDER PROBIOTIKA ZUR BEHANDLUNG VON FETTLEIBIGKEIT
COMPOSITION COMPRENANT PROBIOTIQUES CAPABLE DE SUREXPRIMER LA PROTÉINE CLPB POUR SON UTILISATION DANS LE TRAITEMENT DE L'OBÉSITÉ

(30) Priority: 05.12.2013 EP 13306673; 02.10.2014 EP 14306552
(43) Date of publication of application: 12.10.2016
(73) Proprietor: Institut National de la Santé et de la Recherche Médicale (INSERM), 75013 Paris (FR); Centre Hospitalier Universitaire de Rouen, 76000 Rouen (FR); Universite de Rouen, 76130 Mont-Saint-Aignan (FR)
(72) Inventor: FETISSOV, Serguei, 76183 Rouen (FR); DE, Emmanuelle, 76821 Mont-Saint-Aignan Cedex (FR); TENNOUNE, Naouel, 60000 Beauvais (FR); BRETON, Jonathan, 76183 Rouen Cedex 1 (FR); CHAN-TCHI-SONG, Philippe, 76000 Rouen (FR); DECHELOTTE, Pierre, 76183 Rouen Cedex (FR); LEGRAND, Romain, 76000 Rouen (FR)
(74) Representative: Icosa
(86) International application number: PCT/EP2014/076611
(87) International publication number: WO 2015/082633

(56) References cited:
- TENNOUNE N ET AL: "OP009 COMMENSAL E. COLI INCREASE AFFINITY OF [alpha]-MSH-REACTIVE IMMUNOGLOBULINS AND BODY WEIGHT IN", CLINICAL NUTRITION, vol. 32, 1 September 2013 (2013-09-01), XP028699221, ISSN: 0261-5614, DOI: 10.1016/S0261-5614(13)60011-4
- SINNO M H ET AL: "Regulation of feeding and anxiety by alpha-MSH reactive autoantibodies", PSYCHONEUROENDOCRINOLOGY, OXFORD, GB, vol. 34, no. 1, 1 January 2009 (2009-01-01), pages 140 - 149, XP025815646, ISSN: 0306-4530, [retrieved on 20081219], DOI: 10.1016/J.PSYNEUEN.2008.08.021
- HARROLD J A ET AL: "Altered energy balance causes selective changes in melanocortin-4(MC4-R), but not melanocortin-3 (MC3-R), receptors in specific hypothalamic regions: further evidence that activation of MC4-R is a physiological inhibitor of feeding", DIABETES, AMERICAN DIABETES ASSOCIATION, US, vol. 48, no. 2, 1 February 1999 (1999-02-01), pages 267 - 271, XP002509431, ISSN: 0012-1797, DOI: 10.2337/DIABETES.48.2.267
- TENNOUNE N ET AL: "OP009 COMMENSAL E. COLI INCREASE AFFINITY OF [alpha]-MSH-REACTIVE IMMUNOGLOBULINS AND BODY WEIGHT IN", CLINICAL NUTRITION, vol. 32, 1 September 2013 (2013-09-01), XP028699221, ISSN: 0261-5614, DOI: 10.1016/S0261-5614(13)60011-4
- SINNO M H ET AL: "Regulation of feeding and anxiety by alpha-MSH reactive autoantibodies", PSYCHONEUROENDOCRINOLOGY, OXFORD, GB, vol. 34, no. 1, 1 January 2009 (2009-01-01), pages 140 - 149, XP025815646, ISSN: 0306-4530, [retrieved on 20081219], DOI: 10.1016/J.PSYNEUEN.2008.08.021
- HARROLD J A ET AL: "Altered energy balance causes selective changes in melanocortin-4(MC4-R), but not melanocortin-3 (MC3-R), receptors in specific hypothalamic regions: further evidence that activation of MC4-R is a physiological inhibitor of feeding", DIABETES, AMERICAN DIABETES ASSOCIATION, US, vol. 48, no. 2, 1 February 1999 (1999-02-01), pages 267 - 271, XP002509431, ISSN: 0012-1797, DOI: 10.2337/DIABETES.48.2.267
- MARTIN IANIRE ET AL: "Screening and Evaluation of Small Organic Molecules as CIpB Inhibitors and Antimicrobials", JOURNAL OF MEDICINAL CHEMISTRY, AMERICAN CHEMICAL SOCIETY, US, vol. 56, no. 18, 1 September 2013 (2013-09-01), pages 7177 - 7189, XP009177624, ISSN: 0022-2623
- KENLEY RICHARD A ET AL: "Formoterol fumarate and roxithromycin effects on muscle mass in an animal model of cancer cachexia", ONCOLOGY REPORTS, SPANDIDOS PUBLICATIONS, GR, vol. 19, no. 5, 1 May 2008 (2008-05-01), pages 1113 - 1121, XP009103772, ISSN: 1021-335X
- SAWACKI M ET AL: "275 Anti-cachectic effect of clarithromycin in inoperable non-small cell lung cancer patients", LUNG CANCER, ELSEVIER, AMSTERDAM, NL, vol. 18, 1 August 1997 (1997-08-01), pages 72, XP027323624, ISSN: 0169-5002, [retrieved on 19970801]
- BURNET R ET AL: "Neomycin in resistant obesity", ENDOCRINOLOGIA JAPONICA, JAPAN ENDOCRINE SOCIETY, TOKYO, JP, vol. 22, 1 January 1979 (1979-01-01), pages 4, XP009108651, ISSN: 0013-7219
- SINNO M H ET AL: "Regulation of feeding and anxiety by alpha-MSH reactive autoantibodies", PSYCHONEUROENDOCRINOLOGY, OXFORD, GB, vol. 34, no. 1, 1 January 2009 (2009-01-01), pages 140 - 149, XP025815646, ISSN: 0306-4530, [retrieved on 20081219], DOI: 10.1016/J.PSYNEUEN.2008.08.021
- HARROLD J A ET AL: "Altered energy balance causes selective changes in melanocortin-4(MC4-R), but not melanocortin-3 (MC3-R), receptors in specific hypothalamic regions: further evidence that activation of MC4-R is a physiological inhibitor of feeding", DIABETES, AMERICAN DIABETES ASSOCIATION, US, vol. 48, no. 2, 1 February 1999 (1999-02-01), pages 267 - 271, XP002509431, ISSN: 0012-1797, DOI: 10.2337/DIABETES.48.2.267
- FETISSOV SERGUIEI O ET AL: "Autoantibodies against neuropeptides are associated with psychological traits in eating disorders", PROCEEDINGS OF THE NATIONAL ACADEMY OF SCIENCES, NATIONAL ACADEMY OF SCIENCES, US, vol. 102, no. 41, 11 October 2005 (2005-10-11), pages 14865 - 14870, XP009086395, ISSN: 0027-8424, DOI: 10.1073/PNAS.0507204102
- FETISSOV ET AL: "Autoantibodies against appetite-regulating peptide hormones and neuropeptides: Putative modulation by gut microflora", NUTRITION, ELSEVIER INC, US, vol. 24, no. 4, 8 February 2008 (2008-02-08), pages 348 - 359, XP022552275, ISSN: 0899-9007, DOI: 10.1016/J.NUT.2007.12.006
- KARIN L. MEIBOM ET AL: "The heat-shock protein ClpB of Francisella tularensis is involved in stress tolerance and is required for multiplication in target organs of infected mice", MOLECULAR MICROBIOLOGY, vol. 67, no. 6, 1 March 2008 (2008-03-01), pages 1384 - 1401, XP055044187, ISSN: 0950-382X, DOI: 10.1111/j.1365-2958.2008.06139.x

## Description

The present invention relates to ClpB expressing bacteria and their impact on obesity.

Eating disorders increased all over the world among both men and women over the last 50 years. There is evidence to suggest that in particular individuals in the western world are at the highest risk of developing them and the degree of westernization increases the risk. With recent advances, the scientists understand more and more the central processes of appetite. It is known that interactions between motivational, homeostatic and self-regulatory control processes are involved in eating behavior, which is a key component in eating disorders.

Recent science discovered a further regulator, the human microbiome. Advances of high-throughput DNA sequencing technologies allowed to explore the human microbiome and thus to make a major step towards the understanding of the molecular relationships between the host and its microbiota. This "second genome", the microbiome, has been described in a catalogue of more than 4-5 million, non-redundant microbial putative genes and 1 to 2,000 prevalent bacterial species. Each individual has at least 160 shared species and a number of well-balanced host-microbial molecular relationships that defines groups of individuals.

It is considered that understanding the essential features of symbiotic relationships between microbial communities and their human host may allow to predict host phenotypes, such as health status, from the particular features of indigenous communities.

The composition of gut microbiota for example has been associated with host metabolic phenotypes including obesity and diabetes as well as some neuropsychiatric disorders suggesting that gut bacteria may influence brain-controlled functions and behavior.

In this context, determining the molecular mechanisms linking the microbiota to the host behavior appears, thus, as a necessary step for defining the role of specific microorganisms in host physiology.

The molecular mechanisms at the origin of eating disorders, including anorexia nervosa (AN), bulimia (BN), and binge-eating disorder (BED), are currently unknown. Previous data indicated that immunoglobulins (Igs) or autoantibodies (auto-Abs) reactive with α-melanocyte-stimulating hormone (α-MSH) are involved in regulation of feeding and emotion; however, the origin of such auto-Abs is unknown.

Sinno *et al.* discusses that α-MSH autoAbs affinity extracted from the plasma of rats exposed repeatedly to mild stress, but not from control rats, are able to increase acutely food intake (Sinno et al., "Regulation of feeding and anxiety by α-MSH reactive autoantibodies". Psychoneuroendocrinology. 2009 Jan;34(1):140-149). Furthermore, Harold *et al.* discloses that MC4-R blockade by α-MSH autoAbs may result promoting weight gain (Harold et al., "Altered energy balance causes selective changes in melanocortin-4(MC4-R), but not melanocortin-3 (MC3-R), receptors in specific hypothalamic regions: further evidence that activation of MC4-R is a physiological inhibitor of feeding". Diabetes. 1999 Feb;48(2):267-71).

The inventors discovered, using proteomics, that the CIpB chaperon protein of commensal gut bacteria *E*. *Coli* K12 is a conformational mimetic of α-melanocyte-stimulating hormone (α-MSH), a neuropeptide involved in the regulation of energy metabolism and emotion. Tennoune *et al.* discloses that E. coli K12 increase levels and affinity of α-MSH reactive IgG in healthy Winstar rats, without any significant effect on food intake, suggesting that different properties of α-MSH autoAbs may be relevant to obesity (Tennoune et al., "OP009 Commensal E. coli increase affinity of α-MSH-reactive immunoglobins and body weight in rats", 2013, Clinical Nutrition, vol. 32)
The inventors have shown that ClpB-immunized mice produce anti-ClpB IgG crossreactive with α-MSH, influencing food intake, body weight, anxiety and melanocortin receptor 4 signaling. Furthermore, they show that chronic intragastric delivery of E. coli in mice decreased food intake and stimulated formation of CIpB- and α-MSH-reactive antibodies, while ClpB-deficient E. coli did not affect food intake or antibody levels. Finally, they show that plasma levels of anti-ClpB IgG crossreactive with α-MSH are increased in patients with AN, BN and BED, and that the ED (eating disorders) Inventory-2 scores in eating disorders patients correlate with anti-CIpB IgG and IgM.

As a consequence, they show that the bacterial ClpB protein, which is present in several commensal and pathogenic microorganisms, can be responsible for the production of auto-Abs crossreactive with α-MSH, associated with altered feeding and emotion in humans with eating disorders.

In addition, they show increased plasma concentrations of the ClpB protein in patients suffering from eating disorders.

Therefore, the presence of CIpB protein and/or anti-CIpB antibodies can be related to eating disorders and to a dysregulation of appetite and emotion.

The presence of the ClpB protein and/or anti-ClpB antibodies been correlated with eating disorders and dysregulation of appetite, in return reducing the level of CIpB protein and/or anti-CIpB antibodies can result in regulation of appetite and thus be used as a treatment of eating disorders.

The inventors having showed that intragastric delivery of E. coli in mice decreased food intake and body weight gain due to the presence of ClpB protein in bacteria, the invention further relates to a composition comprising probiotics surexpressing CIpB protein for use in the treatment of obesity.

### Detailed description of the invention

Accordingly, the invention relates to an oral composition comprising probiotics overexpressing ClpB protein for use in the treatment of obesity, said ClpB protein comprising or consisting of an amino acid sequence 80 to 100% identical to the amino acid sequence of SEQ ID NO: 1.

"Obesity" refers herein to a medical condition wherein the subject preferably has a BMI superior to 30.

By "overexpressing" is meant the artificial expression of a protein due to expression of a gene in increased quantity, here the increased expression of the gene encoding for CIpB protein.

Bacteria overexpressing CIpB protein can be prepared by methods known to the skilled person. This can for example be done by bacterial transformation with vectors expressing CIpB DNA.

In one embodiment, the ClpB overexpressing bacterium is selecting from bacteria known as probiotic or commensal non-pathogenic bacteria in humans, e.g. non-pathogenic *Escherichia coli.*

By "effective amount" is meant an amount of bacteria that allows the manifestation of the desired effect. In particular, it is meant an amount of between 1000 million and 10000 million UFC.day⁻¹.

The inventors discovered, using proteomics, that the CIpB chaperon protein of commensal gut bacteria *E.Coli* K12 is a conformational mimetic of α-melanocyte-stimulating hormone (α-MSH), a neuropeptide involved in the regulation of energy metabolism and emotion and that the presence of ClpB expressing bacteria results in dysregulated appetite.

As used herein, "*ClpB* expressing bacterium" refers to bacteria expressing the chaperone protein ClpB.

The "protein disaggregation chaperone", "chaperone protein ClpB", "CIpB protein" or "ClpB" also known as heat shock protein F84.1 is a member of the Hsp100/ClpB family of hexameric AAA+-ATPases. This family comprises bacterial, fungal, and plant Hsp100 ATPases, ClpB being the bacterial representative. As a part of a stress-induced multi-chaperone system, it is involved in the recovery of the cell from heat-induced damage, in cooperation with the Hsp70 system (DnaK, DnaJ and GrpE) in protein disaggregation, a crucial process for cell survival under stress conditions.

During the infection process, bacterial pathogens encounter stress conditions generated by the host defense to eliminate them and respond to this host defense by increasing the synthesis of heat shock and other stress proteins. In this context, ClpB has been described as an essential factor for acquired thermotolerance and for the virulence and infectivity of several Gram-negative and Gram-positive pathogenic bacteria, such as *Staphylococcus aureus, Francisella turalensis, Listeria monocytogenes, Yersinia enterocolitica, and Salmonella thyphimurium.*

In *E.coli* K12 the chaperone protein ClpB also known as heat shock protein F84.1 or htpM and is a protein of 857 amino acids.

Typically, the chaperone protein ClpB comprises or consists of the amino acid sequence of the chaperone protein ClpB from *E*. *Coli* K12 with SEQ ID NO: 1 (NCBI Reference Number: NP_417083.1, as available on November 6, 2013 and/or UniProtKB/Swiss-Prot Number: P63284, as available on November 6, 2013). Preferably, the amino acid sequence of chaperone protein ClpB comprises or consists of an amino acid sequence 80 to 100% identical to the amino acid sequence of SEQ ID NO: 1. Preferably, the amino acid sequence is 90 to 100% identical, more preferably 95 to 100%, most preferably 95, 96, 97, 98, 99 or 100% identical to the amino acid sequence of SEQ ID NO: 1.

Thus, a ClpB expressing bacterium refers to a bacterium expressing or overexpressing the chaperone protein CIpB as defined above or a polypeptide comprising or consisting of an amino acid sequence 80 to 100% identical to the amino acid sequence of SEQ ID NO: 1, more preferably 95 to 100%, most preferably 95, 96, 97, 98, 99 or 100% identical to the amino acid sequence of SEQ ID NO: 1.

By a polypeptide having an amino acid sequence at least, for example, 95% "identical" to a query amino acid sequence of the present invention, it is intended that the amino acid sequence of the subject polypeptide is identical to the query sequence except that the subject polypeptide sequence may include up to five amino acid alterations per each 100 amino acids of the query amino acid sequence. In other words, to obtain a polypeptide having an amino acid sequence at least 95% identical to a query amino acid sequence, up to 5% (5 of 100) of the amino acid residues in the subject sequence may be inserted, deleted, or substituted with another amino acid.

In the context of the present application, the percentage of identity is calculated using a global alignment (*i.e.* the two sequences are compared over their entire length). Methods for comparing the identity of two or more sequences are well known in the art. The « needle » program, which uses the Needleman-Wunsch global alignment algorithm (Needleman and Wunsch, 1970 J. Mol. Biol. 48:443-453) to find the optimum alignment (including gaps) of two sequences when considering their entire length, may for example be used. The needle program is, for example, available on the ebi.ac.uk world wide web site. The percentage of identity in accordance with the invention is preferably calculated using the EMBOSS:: needle (global) program with a "Gap Open" parameter equal to 10.0, a "Gap Extend" parameter equal to 0.5, and a Blosum62 matrix.

Proteins consisting of an amino acid sequence "at least 80%, 85%, 90%, 95%, 96%, 97%, 98% or 99% identical" to a reference sequence may comprise mutations such as deletions, insertions and/or substitutions compared to the reference sequence. In case of substitutions, the protein consisting of an amino acid sequence at least 80%, 85%, 90%, 95%, 96%, 97%, 98% or 99% identical to a reference sequence may correspond to a homologous sequence derived from another species than the reference sequence.

Amino acid substitutions may be conservative or non-conservative. Preferably, substitutions are conservative substitutions, in which one amino acid is substituted for another amino acid with similar structural and/or chemical properties. The substitution preferably corresponds to a conservative substitution as indicated in the table below.

| **Conservative substitutions** | **Type of Amino Acid** |
|---|---|
| Ala, Val, Leu, Ile, Met, Pro, Phe, Trp | Amino acids with aliphatic hydrophobic side chains |
| Ser, Tyr, Asn, Gln, Cys | Amino acids with uncharged but polar side chains |
| Asp, Glu | Amino acids with acidic side chains |
| Lys, Arg, His | Amino acids with basic side chains |
| Gly | Neutral side chain |

ClpB expressing bacteria are well-known from the skilled person and may be identified by any conventional technique.

In one embodiment, the ClpB expressing bacterium is selected from the group constituted of the genus *Staphylococcus aureus, Francisella turalensis, Listeria monocytogenes, Yersinia enterocolitica, Salmonella thyphimurium, Escherichia Coli, Enterobacteriaceae, Shigella sonnei, Shigella flexneri, Shigella dysenteriae, Shigella boydii, Citrobacter youngae, Salmonella bongori* and *Salmonella enterica.*

The CIpB protein comprises two nucleotide binding domains (ATP1 and ATP2) and two oligomerization domains (NBD1 and NBD2). The N-terminal domain might function as a substrate-discriminating domain, recruiting aggregated proteins to the ClpB hexamer and/or stabilizing bound proteins. The NBD2 domain is responsible for oligomerization, whereas the NBD1 domain stabilizes the hexamer probably in an ATP-dependent manner. The movement of the coiled-coil domain is essential for CIpB ability to rescue proteins from an aggregated state, probably by pulling apart large aggregated proteins, which are bound between the coiled-coils motifs of adjacent CIpB subunits in the functional hexamer.

The inventors identified that the CIpB chaperon protein of commensal gut bacteria *E.coli* K12 is a conformational mimetic of α-melanocyte-stimulating hormone (α-MSH), a neuropeptide involved in the regulation of energy metabolism and emotion.

"α-MSH", also known as "α-Melanocyte-stimulating hormone", "alpha-MSH", "α-MSH", alpha-melanotropin, alpha-melanocortin, or alpha-intermedin, is a naturally occurring endogenous peptide hormone of the melanocortin family, with a tridecapeptide structure. The amino acid sequence of α-MSH preferably comprises or consists of the amino acid sequence SYSMEHFRWGKPV (SEQ ID NO: 3) (Gen Pept Sequence ID, PRF: 223274, as available on December 2, 2013). However, there exist three types of alpha-melanocyte-stimulating hormone that differ in their acetyl status, the desacetyl alpha MSH, which lacks an acetyl group; mono-acetyl alpha MSH, in which the amino group of the Ser-1 of SEQ ID NO: 3 is acetylated; and di-acetyl alpha MSH, in which both amino and hydroxy groups of the Ser-1 of SEQ ID NO: 3 are acetylated. α-MSH as used herein refers in particular to the mono-acetylated α-MSH.

It is critically involved in the regulation of energy balance and increasing energy expenditure via activation of the melanocortin receptors type 4 (MC4R), acting both centrally and peripherally. In both humans and mice, plasma α-MSH levels are associated with body weight changes. Furthermore, α-MSH regulates mood and emotion by increasing anxiety.

"Mimetic" refers to a protein that imitates another protein. This imitation is possible since the protein shares certain characteristics with the protein it mimics.

A "conformational mimetic" refers to a protein, that shares at least in part the same conformation as another protein.

The inventors demonstrated that the CIpB protein shares a discontinuous sequence homology between amino acids 542 to 548 from SEQ ID NO: 1 with α-MSH, of amino acid sequence 'RWTGIPV' (referenced under SEQ ID NO: 2). Without being bound by theory, this conformation of CIpB allows stimulating the production of antibodies directed against both CIpB and α-MSH.

Thus "conformational mimetic" herein preferably refers to the capability to stimulate antibody production against ClpB as well as auto antibodies against α-MSH.

In the context of the invention, the term "treating" or "treatment", as used herein, means reversing, alleviating, inhibiting the progress of the disorder to which such term applies, or one or more symptoms of such disorder or condition.

"Preventing" refers to measures taken to prevent the disorder to which such term applies from occurrence or, in early stages of a disorder. Preventing further refers to the inhibition of further development of a disorder to which such term applies.

In the context of the present invention, a "subject" denotes a human or non-human mammal, such as a rodent (rat, mouse, rabbit), a primate (chimpanzee), a feline (cat), or a canine (dog). Preferably, the subject is human. The subject according to the invention may be in particular a male or a female.

Preferably, the subject according to the invention is older than 13.

Preferably, the subject is a female.

By "eating disorder" (ED) is meant psychiatric illnesses defined by criteria from the Diagnostic and Statistical Manual of Mental Disorders 5^{th} Edition (DSM-5) and earlier editions (DSM-4, etc). Abnormal eating habits may involve either insufficient or excessive food intake to the detriment of an individual's physical and mental health. Bulimia nervosa (BN), anorexia nervosa (AN) and binge eating disorder (BED) are the most common specific forms of eating disorders. According to the DSM-5 criteria, to be diagnosed as having anorexia nervosa a person must display: persistent restriction of energy intake leading to significantly low body weight (in context of what is minimally expected for age, sex, developmental trajectory, and physical health), either an intense fear of gaining weight or of becoming fat, or persistent behavior that interferes with weight gain (even though significantly low weight), disturbance in the way one's body weight or shape is experienced, undue influence of body shape and weight on self-evaluation, or persistent lack of recognition of the seriousness of the current low body weight.

According to the DSM-5 criteria, to be diagnosed as having bulimia nervosa a person must display recurrent episodes of binge eating. An episode of binge eating is characterized by both of the following: eating, in a discrete period of time (e.g. within any 2-hour period), an amount of food that is definitely larger than most people would eat during a similar period of time and under similar circumstances and a sense of lack of control over eating during the episode (e.g. a feeling that one cannot stop eating or control what or how much one is eating), recurrent inappropriate compensatory behavior in order to prevent weight gain, such as self-induced vomiting, misuse of laxatives, diuretics, or other medications, fasting, or excessive exercise. The binge eating and inappropriate compensatory behaviors both occur, on average, at least once a week for three months. Self-evaluation is unduly influenced by body shape and weight.

According to the DSM-5 criteria, to be diagnosed as having binge eating disorder a person must display recurrent episodes of binge eating. An episode of binge eating is characterized by both of the following: eating, in a discrete period of time (e.g. within any 2-hour period), an amount of food that is definitely larger than most people would eat during a similar period of time and under similar circumstances, a sense of lack of control over eating during the episode (e.g. a feeling that one cannot stop eating or control what or how much one is eating). The binge eating episodes are associated with three or more of the following:
- eating much more rapidly than normal;
- eating until feeling uncomfortably full;
- eating large amounts of food when not feeling physically hungry;
- eating alone because of feeling embarrassed by how much one is eating;
- feeling disgusted with oneself, depressed or very guilty afterward.

Binge eating occurs, on average, at least once a week for three months.

Other types of eating disorders include Other Specified Feeding or Eating Disorder (OSFED).

According to the DSM-5 criteria, to be diagnosed as having OSFED a person must present with a feeding or eating behaviors that cause clinically significant distress and impairment in areas of functioning, but do not meet the full criteria for any of the other feeding and eating disorders. A diagnosis might then be allocated that specifies a specific reason why the presentation does not meet the specifics of another disorder (e.g. bulimia nervosa- low frequency). The following are further examples for OSFED:
- Atypical anorexia nervosa: all criteria are met, except despite significant weight loss, the individual's weight is within or above the normal range;
- Binge eating disorder (of low frequency and/or limited duration): all of the criteria for BED are met, except at a lower frequency and/or for less than three months;
- Bulimia nervosa (of low frequency and/or limited duration): all of the criteria for bulimia nervosa are met, except that the binge eating and inappropriate compensatory behavior occurs at a lower frequency and/or for less than three months;
- Purging disorder: recurrent purging behavior to influence weight or shape in the absence of binge eating;
- Night eating syndrome: recurrent episodes of night eating, eating after awakening from sleep, or by excessive food consumption after the evening meal. The behavior is not better explained by environmental influences or social norms. The behavior causes significant distress/impairment. The behavior is not better explained by another mental health disorder (e.g. BED);Unspecified Feeding or Eating Disorder (UFED). According to the DSM-5 criteria this category applies to where behaviors cause clinically significant distress/impairment of functioning, but do not meet the full criteria of any of the Feeding or Eating Disorder criteria. This category may be used by clinicians where a clinician chooses not to specify why criteria are not met, including presentations where there may be insufficient information to make a more specific diagnosis (e.g. in emergency room settings).

In the context of the present invention, an eating disorder can thus refer to the above-mentioned list of disorders.

The eating disorder mentioned herein may be anorexia nervosa (AN), bulimia nervosa (BN), binge eating disorder (BED), overeating, hyperphagia, or wasting diseases such as cachexia.

By "appetite" is meant the desire to eat food, felt as hunger. Appetite exists in all higher life-forms, and serves to regulate adequate energy intake to maintain metabolic needs. It is regulated by a close interplay between the digestive tract, energy storage such as in adipose tissue and liver and the brain. Appetite is assessed in a subject by measuring the amount of food ingested and by assessing the subject's desire to eat.

"Dysregulation of appetite" refers to an abnormal appetite which includes increased appetite as well as decreased appetite which is permanently present or reoccurs several times a week and thus contributes to anorexia nervosa, bulimia nervosa, cachexia, wasting disease, overeating, binge eating disorder and hyperphagia.

"Anorexia" relates to the decreased sensation of appetite thus resulting in a reduced appetite. While the term in non-scientific publications is often used interchangeably with anorexia nervosa, many possible causes exist for a decreased appetite, some of which may be harmless, while others indicate a serious clinical condition or pose a significant risk.

"Anorexia nervosa" (AN) refers in the context of the invention to an eating disorder characterized by immoderate food restriction that is characterized by failure to maintain body weight of at least 85% of the normal body weight. Furthermore, the subject suffering from anorexia nervosa has an irrational fear of gaining weight, as well as a distorted body self-perception, where the subject sees him/herself as overweight despite overwhelming evidence to the contrary.

Accordingly, a person suffering from anorexia nervosa may dispose at least one of the psychological traits selected from the group consisting of body dissatisfaction, drive for thinness, perfectionism, ineffectiveness, interpersonal distrust, social insecurity and anhedonia. A person suffering from anorexia nervosa may dispose at least one of the psychological traits selected from the group consisting of body dissatisfaction, drive for thinness and perfectionism. A person suffering from anorexia nervosa may dispose at least one of the psychological traits selected from the group consisting of ineffectiveness, interpersonal distrust, social insecurity and anhedonia.

A subject suffering from anorexia nervosa may have a BMI inferior to 17.

The "BMI" or "body mass index" is defined as the subject's body mass divided by the square of his height. The formulae universally used in medicine produce a unit of measure of kg/m².

"Bulimia" or "Bulimia nervosa" is an eating disorder characterized by binge eating and purging, or consuming a large amount of food in a short amount of time followed by an attempt to rid oneself of the food consumed (purging), typically by vomiting, taking a laxative or diuretic, and/or excessive exercise. Some subjects may tend to alternate between bulimia nervosa and anorexia nervosa. Subject suffering from bulimia may be characterized by a normal BMI range, usually inferior to 25.

"Binge eating disorder" or "BED" refers to an eating disorder characterized by binge eating consisting of eating, in a discrete period of time (e.g. within any 2-hour period), an amount of food that is larger than most people would eat in a similar period of time under similar circumstances, and is accompanied by a feeling of loss of control. The binge eating occurs, on average, at least twice a week for 6 months. Contrary to bulimia the binge eating is not associated with the recurrent use of inappropriate compensatory behavior. Subjects suffering from BED are seriously worried about the binge eating. Furthermore, subjects suffering from BED eat until being physically uncomfortable and nauseated due to the amount of food consumed and/or eat when bored or depressed and/or eat large amounts of food even when not really hungry and/or eat alone during periods of normal eating, owing to feelings of embarrassment about food and/or feel disgusted, depressed, or guilty after binge eating. Subjects with binge eating disorder act impulsively and feel a lack of control over their eating. Furthermore, subject suffering from binge eating disorder have problems coping with stress, anxiety, anger, sadness, boredom and worry.

Subjects suffering of BED may be obese and have a BMI superior to 30.

"Overeating" is the consumption of excess food in relation to the energy that an organism expends (or expels via excretion), leading to weight gaining. Overeating can sometimes be a symptom of binge eating disorder or bulimia. Compulsive over eaters depend on food to comfort themselves when they are stressed, suffering bouts of depression, and have feelings of helplessness.

"Hyperphagia", also known as "polyphagia" refers to excessive hunger (compulsive) or increased appetite. Hyperphagia may be caused by disorders such as Diabetes, Kleine-Levin Syndrome, the genetic disorders Prader-Willi Syndrome and Bardet Biedl Syndrome.

"Wasting disease" refers to the process by which a debilitating disease causes muscle and fat tissue to "waste" away. Wasting can be caused by an extremely low energy intake (e.g., caused by famine), nutrient losses due to infection, or a combination of low intake and high loss.

"Cachexia" is a wasting syndrome associated with loss of weight and/or muscle atrophy and/or fatigue, weakness, and significant loss of appetite which may be caused by cancer, AIDS, chronic obstructive lung disease, multiple sclerosis, congestive heart failure, tuberculosis, familial amyloid polyneuropathy, mercury poisoning (acrodynia) and hormonal deficiency. Cachexia, or wasting, as it may also be called, is seen with several diseases, such as AIDS, cancer, post hip fracture, chronic heart failure, chronic lung disease such as chronic obstructive lung disease (COLD) and/or chronic obstructive pulmonary disease (COPD), liver cirrhosis, renal failure, and autoimmune diseases such as rheumatoid arthritis and systemic lupus, sepsis and severe infection. Furthermore, wasting is also seen in aging

The foremost sign of cachexia is weight loss, not only of fatty tissue but also of muscle tissue and even bone. This non-fatty tissue is also known as "lean body mass."

In addition, there is loss of appetite, weakness (asthenia), and a drop in hemoglobin level (anemia).

Cachexia is found as the terminal state of many different clinical conditions or in chronic diseases such as cancer, infections, AIDS, congestive heart failure, rheumatoid arthritis, tuberculosis, post-hip fracture, cystic fibrosis and Crohn's disease. It can also occur in elderly people who do not have any obvious symptoms of disease.

"Increased appetite" refers to an appetite wherein a subject has a higher food intake than the body requires. This may or may not result in weight gain.

"Normal appetite" thus refers to a subject having a food intake that corresponds to the amount of food the body requires.

"Decreased appetite" and/or "reduced appetite" refers an appetite wherein a subject has a lower food intake than the body requires. This may or may not result in weight loss.

"Food intake" can be measured using a multitude of techniques including self-reporting using e.g. diaries or questionnaires, measurements of calorie-intake from a buffet meal, using weighing of food prior to ingestion, or weighing and analysis of paired quantities of food. The food intake may be measured on a meal basis, a daily basis, a weekly basis or a monthly basis.

In one embodiment the composition is for decreasing weight gain in the subject.

The composition may be used, for example, to decrease appetite, wherein the increased appetite is due to binge eating disorder or overeating.

In an embodiment, the oral composition for use according to the invention results in at least 1% decrease in food intake, such as a decrease of 2%, more preferably 3% or 5% or 7%, and even more preferred 10% below average food intake prior to initiation of treatment.

In another embodiment, the oral composition for use leads to decrease in calorie intake irrespective of changes in food intake, since amount of food ingested may not be directly related to the ingested calorie intake, as the various food items such as fat, carbohydrates and proteins contain different amounts of calories per amount food.

In an embodiment of the invention, the oral composition for use results in a at least 1% decrease in calorie intake, such as a decrease of 2%, more preferably 3%, or 5% or 7%, and even more preferred a decrease of 10% in calorie intake below average calorie intake prior to initiation of treatment.

In one embodiment, the oral composition used in the context of the invention is a pharmaceutical composition.

Pharmaceutical compositions used in the context of the invention are preferably designed to be appropriate for the selected mode or route of administration, and pharmaceutically acceptable diluents, carriers, and/or excipients such as dispersing agents, buffers, surfactants, preservatives, solubilizing agents, isotonicity agents, stabilizing agents, and the like are used as appropriate. Such compositions can be designed in accordance with conventional techniques as disclosed, for example, in Remington, The Science and Practice of Pharmacy, 19th Edition, Gennaro, Ed., Mack Publishing Co., Easton, PA 1995.

The pharmaceutical compositions according to the invention are administered orally in the form of a suitable pharmaceutical unit dosage form. The pharmaceutical compositions of the invention may be prepared in many forms that include tablets, hard or soft gelatin capsules, aqueous solutions, suspensions, and liposomes and other slow-release formulations, such as shaped polymeric gels.

The mode of administration and dosage forms are closely related to the properties of the therapeutic agents or compositions which are desirable and efficacious for the given treatment application..

Pharmaceutical compositions of the invention may be administered by any method known in the art.

Oral liquid pharmaceutical compositions may be in the form of, for example, aqueous or oily suspensions, solutions, emulsions, syrups or elixirs, or may be presented as a dry product for constitution with water or other suitable vehicle before use. Such liquid pharmaceutical compositions may contain conventional additives such as suspending agents, emulsifying agents, non-aqueous vehicles (which may include edible oils), or preservatives.

Pharmaceutical compositions of the invention may also contain excipient such as flavorings, colorings, anti-microbial agents, or preservatives.

The administration regimen may be for instance for a period of at least 2, 3, 4, 5, 6, 7, 8, 9, 10, 15, 20, 25, 30, 35, 40, 45, 50, 55, 60, 65, 70, 75, 80, 85, 90, 95, 100 days.

The dose range depends on the composition to be administered and is defined above.

As is well known in the medical arts, dosages for any one subject depend on many factors, including the patient's size, body surface area, age, the particular compound to be administered, sex, time and route of administration, general health, and other drugs being administered concurrently.

Overweight or too low body weight might also be considered in certain cultures as a physical appearance that is considered to be less attractive.

In one embodiment, an effective amount or a therapeutically effective amount is at least the minimal dose, but less than a toxic dose, of an active agent which is necessary to impart therapeutic benefit or benefit to a subject. Stated another way, such an amount for treating eating disorders, for example, is an amount that induces, ameliorates, or otherwise causes an improvement in the state of the subject, as the regulation of food intake.

By "probiotic" is meant a food additive comprising an effective amount of a microorganism, intended to be introduced in the diet. According to WHO, probiotics are live microorganisms which, when administered in adequate amounts confer a benefit to the host health (WHO, 2001).

ClpB expressing bacteria are well-known from the skilled person and may be identified by any conventional technique.

In one embodiment, the ClpB expressing bacterium in which the expression of the protein is deleted is selected from the group constituted of bacteria known as probiotic or commensal non-pathogenic bacteria in humans, e.g. non-pathogenic *Escherichia coli.*

By "effective amount" is meant an amount of bacteria that allows the manifestation of the desired effect, in the context of the invention, the treatment or prevention of eating disorders. In particular, it is meant an amount of between 1000 million and 10000 million UFC.day ⁻¹.

Throughout the instant application, the term "comprising" is to be interpreted as encompassing all specifically mentioned features as well optional, additional, unspecified ones. As used herein, the use of the term "comprising" also discloses the embodiment wherein no features other than the specifically mentioned features are present (i.e. "consisting of").

The invention will now be described in more detail with reference to the following figures and examples.

### Sequences

SEQ ID NO: 1 shows the amino acid sequence of the chaperon protein ClpB from E. Coli K12 referenced under NCBI Reference Number NP_417083.1 and referenced under Uni-Prot entry P63284.

SEQ ID NO: 2 shows amino acids 542 to 548 of the amino acid sequence of the chaperon protein ClpB from E. Coli K12 of SEQ ID NO: 1.

SEQ ID NO: 3 shows the amino acid sequence of α-MSH from Homo sapiens referenced under Gen Pept Sequence ID, PRF: 223274.

SEQ ID NO: 4 shows the nucleic acid sequence of the nucleotide primers CIpB.

SEQ ID NO: 5 shows the nucleic acid sequence of the nucleotide primers CIpB.

### Figures

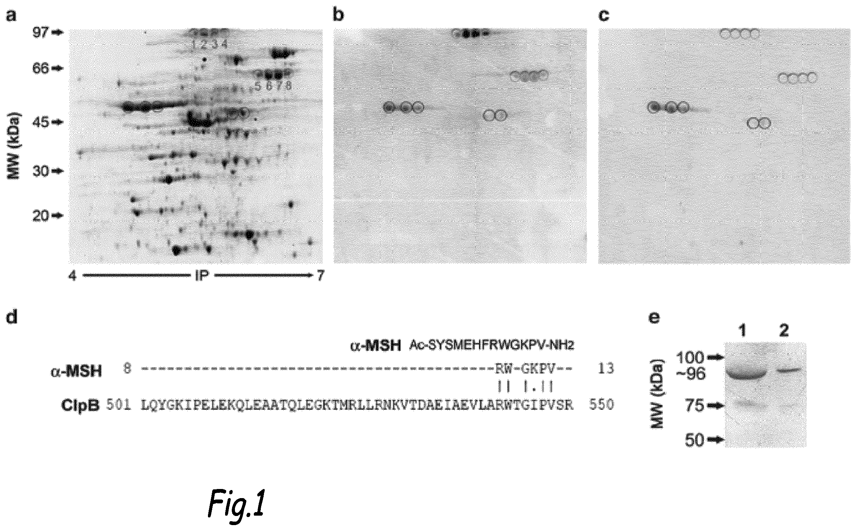

**Figure 1****: Proteomic identification of molecular mimicry between E. coli K12 proteins and α-MSH.**
   **(a)** 2D GE of E. coli cytoplasmic proteins. **(b, c)** Immunoblots of E. coli proteins detected with Rb anti-α-MSH IgG, preadsorbed (c) or not (b) with α-MSH. Circles in red surround the spots specifically recognized by α-MSH IgG which were used for protein identification. Circles in blue indicate nonspecific spots. Proteins identified in the spots 1-4 are isoforms of ClpB. **(d)** α-MSH and CIpB amino-acid sequence alignments using the Stretcher program. **(e)** Western blot of the recombinant ClpB, revealed with anti-α-MSH IgG. Lanes 1 and 2, 20 and 10 µg of ClpB, respectively.

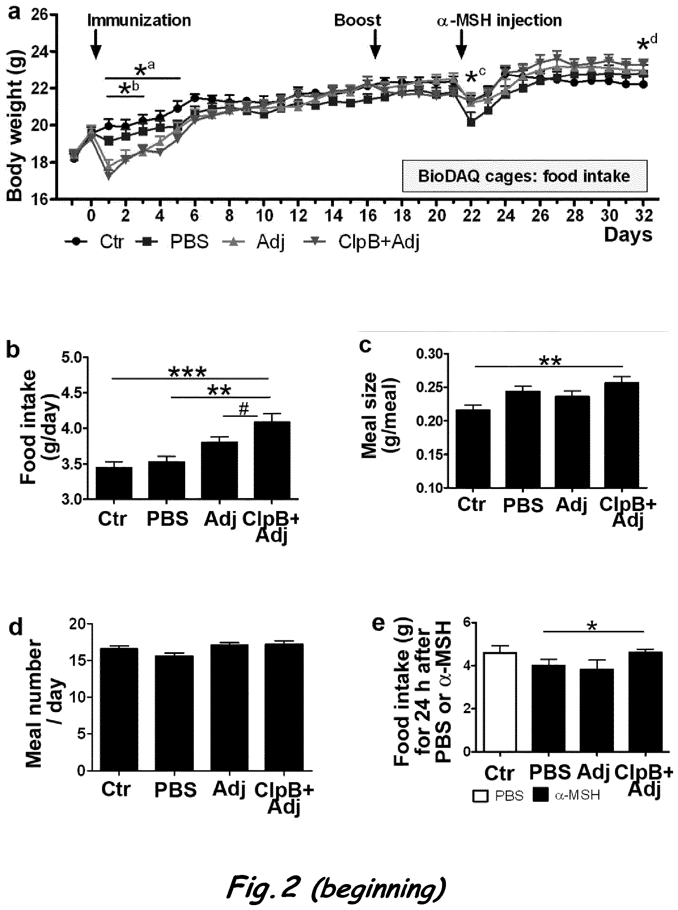

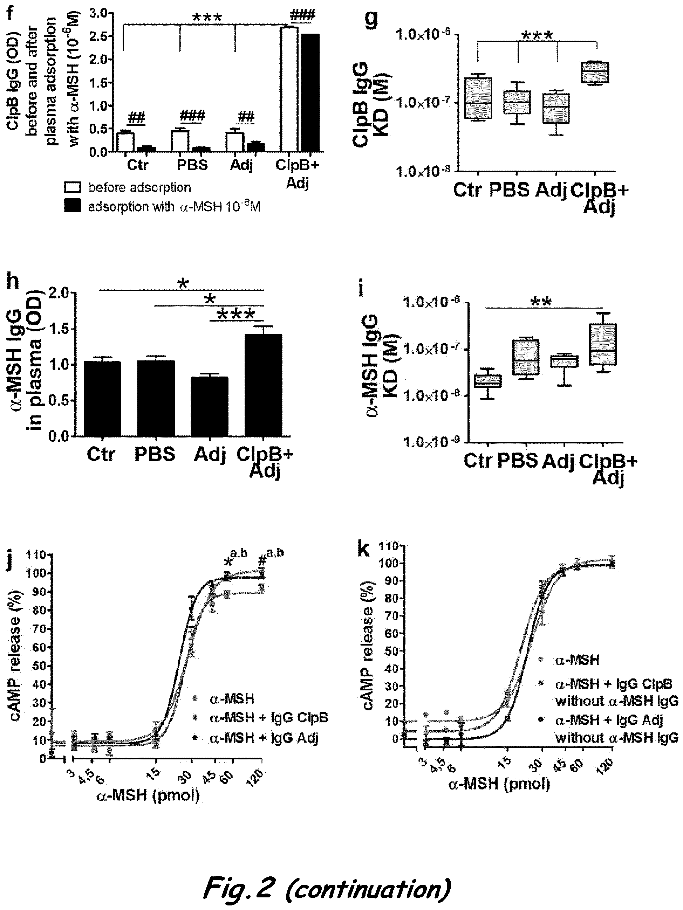

**Figure 2****. ClpB immunization in mice.**
   ClpB-immunized mice (ClpB+Adj) were compared with mice receiving adjuvant (Adj), PBS or controls (Ctr). **(a)** Body weight changes during 32 days of the study. Food intake and feeding pattern were studied during the last 2 weeks in the BioDAQ cages. Mean daily food intake **(b)**, meal size **(c)** and meal number **(d)** during last 10 days of the study. **(e)** Food intake during 24 h after injection of α-MSH (100 µg kg- 1 body weight, i.p.) or PBS. **(f)** Plasma levels of CIpB-reactive IgG before and after adsorption with 10⁻⁶M α-MSH. **(g)** Affinity of anti-ClpB IgG shown as the dissociation equilibrium constants (KD values). **(h)** Plasma levels of α-MSH-reactive total IgG. **(i)** Affinity (KD) of anti-α-MSH IgG. **(j)** cAMP assay in human embryonic kidney-293 cells overexpressing MC4R after stimulation by α-MSH alone or together with IgG (0.5 mg ml⁻¹) pooled from ClpB-immunized or from Adj-injected mice. (k) The cAMP assay was performed with IgG depleted from anti-α-MSH IgG. **(a)** Two-way repeated measurement analysis of variance (ANOVA) before α-MSH injection (100 µg kg⁻¹ body weight, i.p.), Po0.0001, Bonferroni post tests *a at least, Po0.05 CIpB group vs Ctr; *b at least, Po0.05 Adj group vs Ctr.; *c, Po0.05, Student's t-test CIpB group vs PBS; and *d, Po0.05, Student's t-test CIpB group vs Ctr. **(b)** ANOVA P=0.0002, Tukey's post tests ***Po0.001, **Po0.01, #Po0.05, Student's t-test. **(c)** ANOVA P=0.007, Tukey's post tests **Po0.01. **(e)** Student's t-test, *Po0.05. **(f, g)** ANOVA Po0.0001, Tukey's posttests ***Po0.001 CIpB +Adj vs other groups, paired t-test ##Po0.01, ###Po0.001. **(h)** ANOVA P =0.0002, Tukey's post tests ***Po0.001, *Po0.05; **(i)** Kruskal-Wallis test P =0.003, Dunn's post test **Po0.01, (mean ±s.e.m., n=8). **(j)** ANOVA P= 0.005, Tukey's post test *Po0.05; ANOVA P =0.04, Student's t-test #Po0.05, aClpB vs α-MSH, bClpB vs Adj. (mean ± s.e.m.; j, n =6, k, n=3).

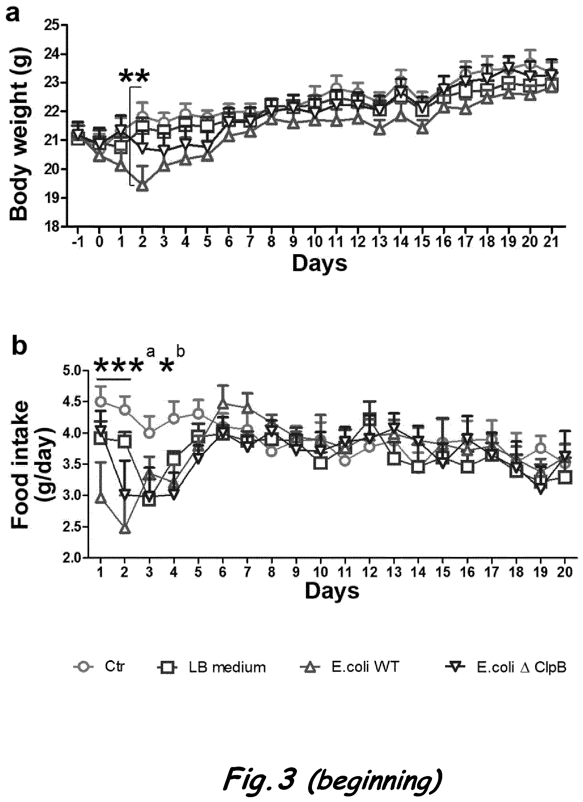

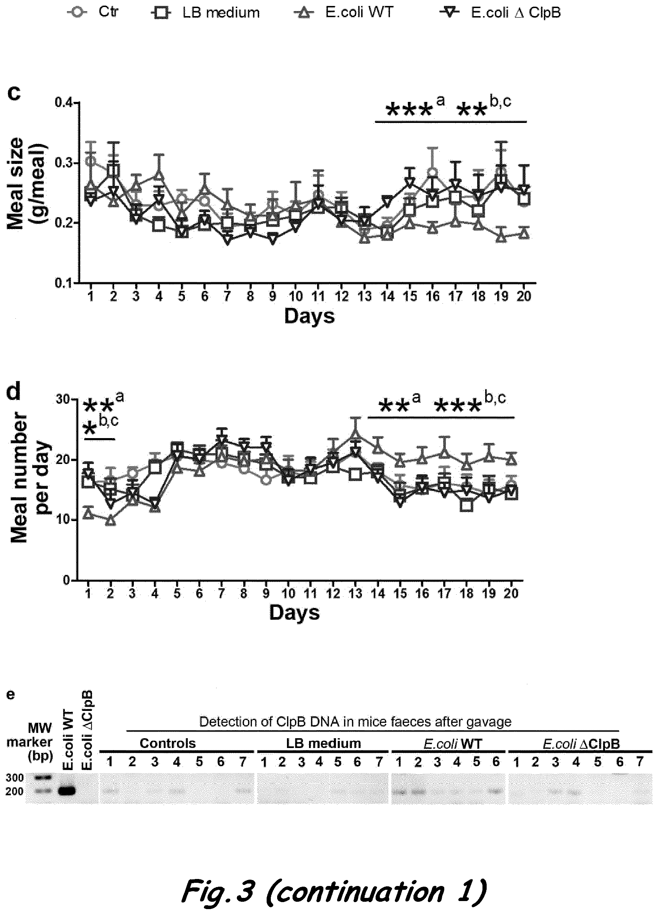

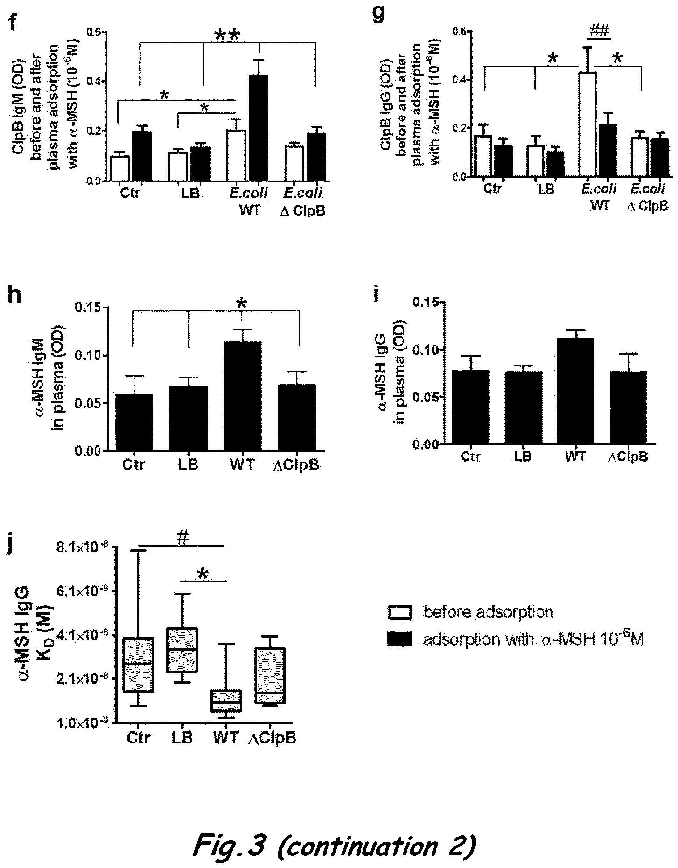

**Figure 3****: E. coli supplementation in mice.**
   Effects of intragastric daily gavage (days 1-21) in mice with either E. coli K12 wild-type (WT), CIpBdeficient (ΔClpB) E. coli K12 or LB medium on body weight **(a)**, food intake **(b)**, meal size **(c)** and meal number **(d). (e)** PCR detection of a 180-base pair fragment of the bacterial ClpB DNA, first lane, molecular weight marker, second lane DNA from in vitro cultures of E. coli K12 WT, third lane DNA from in vitro cultures of E. coli K12 ΔClpB, and the remaining lanes DNA from mice feces collected at day 21. Plasma levels in optical density in enzyme-linked immunosorbent assay of anti-CIpB IgM **(f)** and IgG **(g)** before and after adsorption with 10⁻⁶M α-MSH.
   Plasma levels of anti-α-MSH IgM **(h)** and IgG **(i). (j)** Affinity (equilibrium constant) of anti-α-MSH IgG. **(a)** Two-way repeated measurements analysis of variance (ANOVA), P =0.3, Bonferroni post test day 2, **Po0.01 control (Ctr) vs E. coli WT. **(b)** ANOVA days 1-2, P =0.0006, Tukey's post tests ***Po0.001, *Po0.05, E. coli WT vs aCtr and bLB. **(c)** Kruskal-Wallis (K-W) test third week P=0.0001, Dunn's post tests, ***Po0.001, **Po0.01, E. coli WT vs aCtr, bLB and CΔClpB. **(d)** ANOVA days 1-2, P =0.006, Tukey's post tests **Po0.01, *Po0.05, K-W test third week Po0.0001, Dunn's post tests, ***Po0.001, **Po0.01, E. coli WT vs aCtr, bLB and CΔClpB. **(f)** K-W test, before adsorption P =0.02, Dunn's post tests *Po0.05, ANOVA after adsorption, Po0.0001, Tukey's post tests **Po0.01, E. coli WT vs other groups. **(g)** ANOVA before adsorption, P=0.01, Tukey's post tests *Po0.05, E. coli WT vs other groups, paired t-test ##Po0.01. **(h)** Student's t-test, E. coli WT vs other groups *Po0.05. **(j)** K-W test P =0.02, Dunn's post test *Po0.05, Mann-Whitney test, #Po0.05. (mean ± s.e.m., n=8).

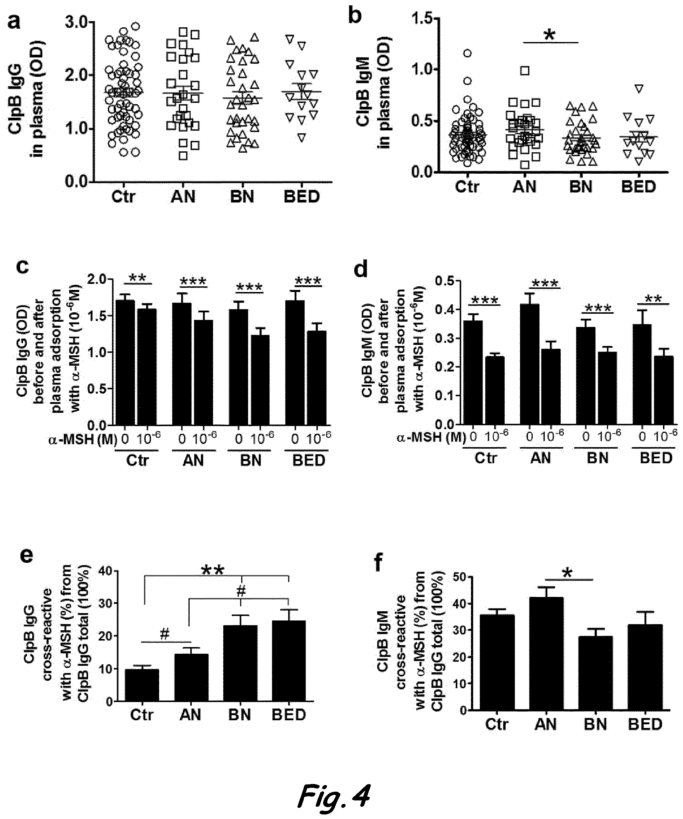

**Figure 4****. Anti-CIpB antibodies in ED patients.**
   Plasma levels of anti-ClpB IgG **(a)** and IgM **(b)** in healthy women (control, Ctr) and in patients with AN, BN and BED. Plasma levels of CIpB IgG **(c)** and IgM **(d)** before and after adsorption with 10⁻⁶M α-MSH.Percentage of α-MSH crossreactive anti-CIpB IgG **(e)** and IgM **(f). (b)** Student's t-test *Po0.05. **(c, d)** Paired t-tests, ***Po0.001, **Po0.01. **(e)** Kruskal-Wallis test Po0.0001, Dunn's post test, **Po0.01, Mann-Whitney test #Po0.05. **(f)** Analysis of variance P =0.02, Tukey's post test *Po0.05. (mean ±s.e.m., Ctr, n =65, AN, n=27 BN, n =32 and BED, n=14).

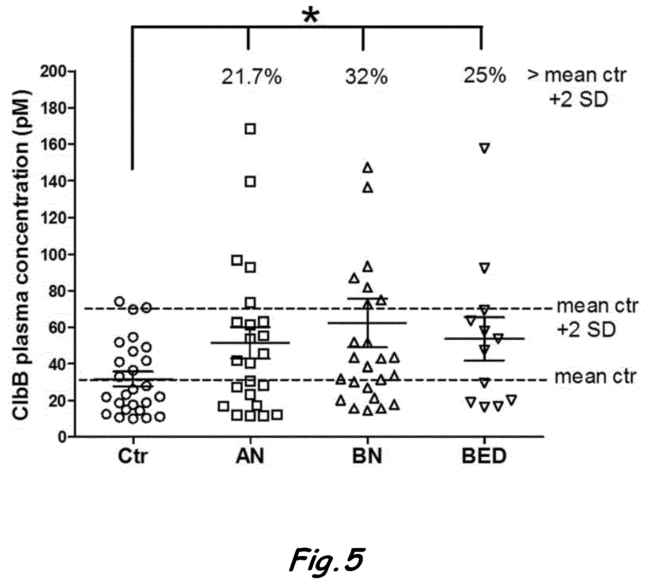

**Figure 5****. Plasma concentrations of bacterial ClpB protein in patients with eating disorders and healthy controls (Ctr).**
   AN, anorexia nervosa, BN, bulimia nervosa, BED, binge-eating disorder. *p<0,05 Student's t-test of mean CIpB concentrations vs. Ctr. Percentage (%) of patients having ClpB concentrations higher than mean+2 standard deviations (SD) of controls.

### Examples

The following examples demonstrate that the CIpB chaperon protein of commensal gut bacteria E. *Coli* K12 is a conformational mimetic of α-melanocyte-stimulating hormone (α-MSH), a neuropeptide involved in the regulation of energy metabolism and emotion. They also reveal a molecular link between ClpB expressing gut bacteria and the regulation of motivated behavior and emotion via production of ClpB protein and anti-ClpB antibodies crossreactive with α-MSH. They further support the involvement of CIpB-expressing microorganisms in increased ClpB protein and ClpB antibody production and establishment of abnormal feeding behavior and emotion.

### Example 1

### Materials and Methods

### E.coli K12 culture and protein extraction

The bacterial strain used in this study was E. coli K12, provided by UMR 6270 CNRS Laboratory in Rouen University, France. E. coli K12 was grown in 250 ml Luria Bertani (LB) broth (MP Biomedicals, Illkirch, France) at 37 °C for 24 h. Protein extraction was performed as described by Marti et al. (PLoS ONE 2011, e26030). In brief, bacteria were harvested by centrifugation at 4000 g for 30 min at 4 °C and the resulting pellet was resuspended in extraction buffer (300mM NaCl and 20mM Tris-HCl, pH 8). The suspension was disrupted by sonication (3 × 3 min, pulse ON 1 s, OFF 1 s at 21% of amplitude) and centrifuged at 10 000 g for 10 min at 4 °C. The supernatant was recovered and ultracentrifuged at 4 °C for 45 min at 60 000 g to further separate proteins into cytoplasmic (supernatant) and envelope (pellet) fractions. Protein concentrations were measured using 2-D Quant Kit (GE Healthcare, Piscataway, NJ, USA).

### Two-dimensional polyacrylamide gel electrophoresis

For two-dimensional (2D) polyacrylamide gel electrophoresis (PAGE), 400 µg of E. coli K12 protein extract were added to iso-electro focusing buffer (7M urea, 2M thiourea and 0.5% ampholytes, pH 4-7, 20mM DTT, 2mM TBP, 2% CHAPS and 0.005% bromophenol blue) and solubilized for 60 min at room temperature with slight shaking. The first-dimensional gel separation was carried out using ReadyStrip IPG Strip (18 cm, pH 4-7 NL, Bio-Rad, Marnes-la-Coquette, France). After 24 h of passive rehydration of the strip with iso-electro focusing buffer, the protein sample was added to the strips through a loading cup placed at 1.5 cm from the cathode. Isoelectro focusing was performed with the Ettan IPGphor 3 System (GE Healthcare, Orsay, France) in four steps (31 500 Vh): 500 V for 1 h, 1000 V gradient, 10 000 V gradient and 10 000 V for 2 h. After two equilibration steps with 2% DTT and 2.5% iodoacetamide, respectively, the second dimension, that is, a SDS-PAGE, (10% polyacrylamide gel, 20 cm × 18- cm × 1 mm) was performed on an Ettan Daltsix vertical electrophoresis system (GE Healthcare) with 12 mA per gel. After SDS-PAGE, the 2D gel was fixed for 2 h in 2% (vol:vol) orthophosphoric acid and in 50% (vol:vol) methanol at room temperature. Gels were then rinsed with water, and the protein spots were visualized by CBB G-250 (Bio-Rad) staining (34% (vol: vol) methanol, 17% (wt:vol) ammonium sulfate, 2% (vol:vol) orthophosphoric acid and 0.66 g CBB G-250 per liter).

### Immunoblotting

Following 2D-PAGE, E. coli cytoplasmic proteins were transferred onto Hybond-ECL PVDF membrane (GE Healthcare) via a dry transfer method (Trans Blot Cell, Bio-Rad, USA) and a constant current of 0.8 mA.cm⁻² of the membrane size for 2 h. After transfer, membranes were blocked with 5% (wt:vol) milk (Regilait, France) in phosphate-buffered saline (PBS; 10 mmol.l⁻¹ Tris, pH 8, and 150mM.I⁻¹ NaCl) plus 0.05% (vol:vol) Tween 20. After washes, membranes were incubated overnight at 4 °C with polyclonal rabbit anti-α-MSH IgG (1:1000, Peninsula Laboratories, San Carlos, CA, USA), followed by washes and incubation with polyclonal swine anti-rabbit horseradish peroxidase-conjugated Igs (1:3000; Dako, Trappes, France). Immunoblots were revealed by the ECL detection system (GE Healthcare) and were scanned with ImageScanner II (GE Healthcare) previously calibrated by using a greyscale marker (Kodak) and digitalized with Labscan 6.00 software (GE Healthcare). The same procedure was performed after adsorption of rabbit anti-α-MSH IgG with 10⁻⁶M of α-MSH peptide (Bachem AG, Bubendorf, Switzerland) overnight at 4 °C.

### Protein identification

The protein spots of interest were excised from CBB G-250-stained 2D gels using the Ettan Spot Picker (GE Healthcare), and automated in-gel digestion of proteins was performed on the Ettan Digester (GE Healthcare). Protein extracts were then resuspended in 10 µl of 5% (vol:vol) acetonitrile/0.1% (vol:vol) formic acid and then analyzed with a nano-LC1200 system coupled to a 6340 Ion Trap mass spectrometer equipped with a nanospray source and an HPLC-chip cube interface (Agilent Technologies, Courtaboeuf, France). In brief, peptides were enriched and desalted on a 40-nl RPC18 trap column and separated on a Zorbax (30-nm pore size, 5-µm) particle size) C18 column (43mm long × 75 µm inner diameter; Agilent Technologies). A 9-min linear gradient (3-80% acetonitrile in 0.1% formic acid) at a flow rate of 400 nl.min⁻¹ was used, and the eluent was analyzed with an Ion Trap mass spectrometer. For protein identification, MS/MS peak lists were extracted and compared with the protein databases by using the MASCOT Daemon version 2.2.2 (Matrix Science) search engine. The searches were performed with the following specific parameters: enzyme specificity, trypsin; one missed cleavage permitted; no fixed modifications; variable modifications, methionine oxidation, cysteine carbamidomethylation, serine, tyrosine and threonine phosphorylation; monoisotopic; peptide charge, 2+ and 3+; mass tolerance for precursor ions, 1.5 Da; mass tolerance for fragmentations, 0.6 Da; ESI-TRAP as instrument; taxonomy, E. coli; National Center for Biotechnology Information (NCBI) database (NCBlnr 20120531 (18280215 sequences, 6265275233 residues); Bethesda, MD, USA). Protein hits were automatically validated if they satisfied one of the following criteria: identification with at least two top-ranking peptides (bold and red) each with a MASCOT score of 454 (Po0.01), or at least two top-ranking peptides each with a MASCOT score of 447 (Po0.05). To evaluate false-positive rates, all the initial database searches were performed using the 'decoy' option of MASCOT. Results were considered relevant if the false-positive rate never exceeded 1%.

### Protein identification from OFFGEL

High-resolution E. coli K12 protein separation into 24 fractions was done onto the 3100 OFFGEL fractionator using the OFFGEL pH3-10 kit (Agilent Technologies). Protein samples (400 µg) preparation and assembly of all parts of the OFFGEL systems were done according to the procedures described in the Agilent Quick start Guide. OFFGEL fractionation was performed using the standard program OG24PRO with maximum limited current parameters (8000 V, 50 µA and 200 mW) until 64 KVh was reached after 30 h. At the end of the experiment, all fractions were transferred into a 0.8-ml deep well (Thermo Fisher Scientific, Illkirch, France) and stored at - 20 °C. Nine protein-containing fractions recovered from the central part of the OFFGEL were studied by western blot using rabbit anti-α-MSH IgG (Peninsula Laboratories) followed by protein identification as described above.

### Immunization and behavior in mice

All experimental protocols were conducted according to US National Institutes of Health guidelines and EU directives, and animal experiments were approved by the Institutional Ethical Committees. Two-month-old male C57BI6 mice (Janvier Laboratories, L'Arbresle, France) were acclimated to the animal facility for 1 week with 12 h light-dark cycle, lights on at 0700 hours and were kept in standard mouse-holding cages (n = 8) each. Mice were fed ad libitum with standard pelleted rodent chow (RM1 diet, SDS, UK) with drinking water always available and were manipulated daily by gentle holding and measuring body weight. During acclimatization, mice were distributed between four cages to obtain the similar mean body weight per mouse per cage. After 1 week of acclimatization, mice from each cage were assigned to one of four study group and received following treatments: (i) Group 1, ClpB immunization (n = 8): ClpB protein (Delphi Genetics, Gosselies, Belgium) 50 µg per mouse in 200 µl of 1:1 (vol:vol) of PBS with Complete Freund's Adjuvant (Sigma, St Louis, MO, USA), intraperitoneally (i.p.); (ii) Group 2, adjuvant injection controls (n = 8): 200 µl of Complete Freund's Adjuvant in PBS (1:1 (vol:vol), i.p.); (iii) Group 3, PBS injection controls (n = 8): 200 µl of PBS (i.p.); and (iv) Group 4, intact controls (n = 8): received no injections, and then all mice were returned to their holding cages. Fifteen days later, mice were given a boost immunization and the following treatments: (i) Group 1 (n = 8), CIpB protein (Delphi Genetics) 50 µg per mouse in 200 µl of 1:1 (vol:vol) of PBS with Incomplete Freund's Adjuvant (Sigma) i.p.; (ii) Group 2 (n = 8): 200 µl of Incomplete Freund's Adjuvant in PBS (1:1 (vol:vol), i.p.); (iii) Group 3, (n = 8): 200 µl of PBS (i.p.); and iv) Group 4, (n = 8): received no injections. Next day after the boost, mice were placed individually into the BioDAQ mouse cages (Research Diets, Inc., New Brunswick, NJ, USA) each equipped with an automatic feeding monitor. Food (Serlab, Montataire France) and drinking water were available ad libitum and body weight was measured daily. After 3 days of acclimatization to the BioDAQ cages, mice received the following treatments: Groups 1, 2 and 3 (each n = 8), that is, mice that have been immunized with ClpB, injected with adjuvants and with PBS, respectively, all received an acute injection of α-MSH peptide (Bachem AG), 100 µg.kg⁻¹ body weight in 100 µl of PBS (i.p.) at 1000 hours. The control mice (n = 8) received PBS only (i.p.). Feeding data was continuously monitored and analyzed using the BioDAQ data viewer 2.3.07 (Research Diets). For the meal pattern analysis, the inter-meal interval was set at 300 s.

After the feeding study, mice were placed in individual mouse-holding cages with food and water available ad libitum, and were analyzed for locomotor activity and anxiety in O-maze (Med Associate, Inc., St Albans, VT, USA) tests performed during 2 consecutive days. Two hours after the O-maze test, mice were killed by decapitation in a guillotine and trunk blood was collected into EDTA-containing tubes. Plasma was separated by centrifugation at 3500 r.p.m. (1.4 g) for 10 min at 4 °C and stored at - 80 °C before assay.

### Locomotor activity and anxiety tests

After feeding study in the BioDAQ cages, mice were analyzed for locomotor activity using a Versamax Animal Activity Monitor (AccuScan Instruments, Inc., Columbus, OH, USA). Next day after the locomotor activity test, all mice were tested for their anxiety in an elevated O-maze. The elevated-O-maze is a variation of more commonly used elevated plusmaze pharmacologically validated for anxiety testing in rodents. The advantage of the O-maze is that it lacks the ambiguous central square of the traditional plus-maze. The O-maze consisted of a circular infrared platform (outer diameter 120 cm) elevated 80 cm above the floor, featuring two open and two closed segments made of gray plastic. The closed segments were enclosed by walls extending 20 cm above the surface of the maze and covered with a black infrared plexiglas lid. Each test started by placing the mouse into one of the two closed segments. The test lasted 5 min and was recorded using a video camera placed above the O-maze and the EthoVision video tracking software (Noldus IT, Wageningen, The Netherlands). Measurements of distance and time spent in the open and closed segments were analyzed. Between each mouse tests, the O-maze was cleaned with 30% ethanol.

### ClpB and α-MSH autoantibody assay

Plasma levels of auto-Abs reacting with ClpB, α-MSH and adrenocorticotropic hormone were measured using enzyme-linked immunosorbent assay according to a published protocol (Fetissov SO., Methods Biol Mol 2011). In brief, CIpB protein (Delphi Genetics), α-MSH or adrenocorticotropic hormone peptides (Bachem AG) were coated onto 96-well Maxisorp plates (Nunc, Rochester, NY, USA) using 100 µl and a concentration of 2 µg.ml⁻¹ in 100mM NaHCO3 buffer, pH 9.6, for 72 h at 4 °C. Plates were washed (5 min for three times) in PBS with 0.05% Tween 200, pH 7.4, and then incubated overnight at 4 °C with 100 µl of mouse plasma diluted 1:200 in PBS to determine free auto-Ab levels or diluted 1:200 in dissociative 3M NaCl and 1.5M glycine buffer, pH 8.9, to determine total auto-Ab levels. The plates were washed (three times) and incubated with 100 µl of alkaline phosphatase (AP)-conjugated goat antimouse IgG (1:2000) or anti-mouse IgM (1:1000), all obtained from Jackson ImmunoResearch Laboratories, Inc. (West Grove, PA, USA). Following washing (three times), 100 µl of p-nitrophenyl phosphate solution (Sigma) was added as AP substrate. After 40 min of incubation at room temperature, the reaction was stopped by adding 3 N NaOH. The optical density was determined at 405 nm using a microplate reader Metertech 960 (Metertech Inc., Taipei, Taiwan). Blank optical density values resulting from the reading of plates without addition of plasma samples were subtracted from the sample optical density values. Each determination was done in duplicate. The variation between duplicate values was inferior to 5%. Similar protocol was used to measure anti-ClpB IgG and IgM in human plasma (1:400) using corresponding anti-human IgG or IgM AP-conjugated antibodies (1:2000, Jackson ImmunoResearch Laboratories, Inc.).

### Absorptions of ClpB antibodies with α-MSH

Plasma samples of mice, diluted 1:200 in PBS, or humans, diluted 1:400 in PBS, were preincubated with 10⁻⁶M α-MSH peptide (Bachem AG) overnight at 4 °C before adding the samples to 96-well Maxisorp plates (Nunc) coated with CIpB protein (Delphi Genetics). IgG and IgM antibodies reactive with ClpB were detected by enzyme-linked immunosorbent assay using corresponding anti-mouse or anti-human AP-conjugated antibodies (Jackson ImmunoResearch Laboratories, Inc.) as described above. Percentage of ClpB antibodies crossreactive with α-MSH were calculated relative to levels of anti-ClpB antibodies detected without absorption in each individual plasma sample equal 100%.

### IgG purification from plasma

IgG purification and affinity assay were performed according to a published protocol (Legrand et al., Protoc Exch 2014, doi:10.1038/protex2014.004). Extraction of plasma globulins was done by plasma acidification and separation on C18 SEP column (Phoenix Pharmaceuticals, Burlingame, CA, USA), then 500 µl of mouse plasma was mixed with 500 µl of buffer A (1% trifluoroacetic acid in water). The column was activated in 1 ml of buffer B (60% acetonitrile in 1% trifluoroacetic acid) by 3 min centrifugation with 700 r.p.m. and rinsed three times with 3 ml of buffer A. Diluted plasma (1:1 in buffer A) was added to the column and the effluent (1 ml) was saved (frozen at - 20 °C) for further purification of IgG. Total IgG were purified from the effluents of mouse plasma samples using the Melon Gel Kit (Thermo Fisher Scientific, Rockford, IL, USA). Plasma effluents diluted 1:4 in kit's purification buffer was added on washed melon gel deposited in a column. Column was spun 1 min at 6000 r.p.m., and the IgG containing effluent was saved and frozen at - 20 °C before lyophilization. Lyophilized IgG were reconstituted in the HBS-EP buffer (GE Healthcare, Piscataway, NJ, USA). For the cyclic adenosine monophosphate (cAMP) experiment, IgG purified from eight mice of the CIpB and of the adjuvant control group were combined, respectively, into two pools that were divided in two parts. One part was used directly in cAMP assay and the other was further purified using affinity chromatography for α-MSH (Bachem AG) coated on activated UltraLink beads (Pierce, Rockford, IL, USA). The α-MSH IgGdepleted IgG effluents were saved, lyophilized and diluted in PBS.

### Affinity kinetics assay

Affinity kinetics of mouse IgG for ClpB and α-MSH was determined by a biospecific interaction analysis based on the surface plasmon resonance phenomenon on a BIAcore 1000 instrument (GE Healthcare). α-MSH (Bachem AG) or CIpB protein (Delphi Genetics) were diluted to 0.5 mg.ml⁻¹ in 10mM sodium acetate buffer, pH 5.0 (GE Healthcare), and were covalently coupled on the sensor chips CM5 (GE Healthcare) by using the amine coupling kit (GE Healthcare). All measures were performed on the same α-MSH or ClpB-coated chips. For the affinity kinetic analysis, a multicycle method was run with five serial dilutions of each IgG sample: 3360, 1680, 840, 420 and 210 (nmol), including a duplicate of 840 nmol and a blank sample (HBS-EP buffer only). Each cycle included 2 min of analyte injection and 5 min of dissociation with flow speed 30 µl.min⁻¹ at 25 °C.

Between injections of each sample, the binding surface was regenerated with 10mM NaOH, resulting in the same baseline level of the sensorgram. The affinity kinetic data were analyzed using BiaEvaluation 4.1.1 program (GE Healthcare). For fitting kinetic data, the Langmuir's 1:1 model was used, and the sample values were corrected by subtracting the blank values.

### In vitro cAMP assay

Stable cell line of human embryonic kidney-293 cells expressing human MC4R was generated using a lentiviral transduction technology and purchased from Amsbio (Oxon, UK). High expression of MC4R mRNA in transfected cells was validated by reverse transcription PCR in Amsbio and in our laboratory. The presence of the transgene in cells before each experiment was verified by the visualization at a fluorescence microscope of the green fluorescent protein, which gene was inserted in the same with MC4R lentivector but under a different promoter. The α-MSH peptide (Bachem AG) was diluted in the induction buffer: PBS, 500 µM IBMX, 100 µM RO 20-1724 (Sigma), 20mM MgCl2 to the final concentrations of 2, 1, 750, 500, 250, 100, 75, 50 and 10 nM corresponding to the α-MSH doses of 0.6, 3, 4.5, 6, 15, 30, 45, 60 and 120 pmol, respectively, and also included one blank sample. After unfreezing, the cells were cultured in 250 ml tissue culture flasks (BD-Falcon, Beckton-Dickinson, Bedford, MA, USA) in Dulbecco's modified Eagle medium 4.5 g.l⁻¹ glucose (Eurobio, Courtaboeuf, France) supplemented with (2mM L-glutamine, 10% fetal calf serum, 0.1mM nonessential amino acids and 1% penicillin-streptavidin) in humidified cell culture incubator at 37°C, 5% CO2 for 8-10 days. At the day of experiment, cultured MC4R human embryonic kidney-293 cells were treated with 0.25% trypsin-EDTA (Sigma-Aldrich) and cell pellets were resuspended in PBS to obtain 5000 cells per well (10 µl) in a nontreated bioluminescence white 96-microwell plate (Nunc, Roskilde, Denmark). The cAMP production was measured using the bioluminescent assay cAMP-Glo Max Assay kit (Promega, Madison, Wl, USA) according to the manufacturer's instructions. In brief, the cells were incubated for 15 min at room temperature with different concentrations of α-MSH peptide alone or α-MSH together with mouse IgG pools from ClpB-immunized or adjuvant control groups, and which were added to the cells just before α-MSH. Serial dilutions of cAMP standard (provided by the kit) were assayed on the same microplate. cAMP detection solution was added to each well, then the cells were homogenized by agitation and centrifuged 2 min at 1000 r.p.m. and then incubated for 20 min at 23°C. Kinase-Glo reagent substrate was added in each well and after 10 min of incubation at 23°C, the luminescence was read with a bioluminescence instrument (Safas Spectrometer, Monaco). Three tests for each dilution were performed in separate wells and were repeated at two separate days resulting in n = 6 for each point of the cAMP activation curve when native IgG were used. After depletion of native IgG from anti-α-MSH IgG fraction, the same experiment was performed with each α-MSH concentration and IgG as described above.

### E. coli gavage in mice

One-month-old male C57BI6 mice (Janvier Laboratories) were acclimated to the animal facility for 1 week and maintained as described above. Mice were distributed into four groups (n = 8 in each) as follows: (i) gavaged with 108 E. coli K12 bacteria; (ii) gavaged with 108 E. coli K12 bacteria deficient for ClpB; (iii) gavaged with LB medium only; and (iv) controls that did not receive any treatments. The ClpB mutant strain was generated in the Bernd Bukau's Laboratory (ZMBH, Heidelberg University, Heidelberg, Germany) and was kindly provided together with the corresponding wildtype (WT) E. coli bacteria by Dr Axel Mogk. Mice were placed individually into the BioDAQ cages (Research Diets) and intragastrically gavaged daily before the onset of dark phase for 21 days with 0.5 ml of LB medium with or without bacteria. During the last day of gavage, mice feces were collected and frozen. After gavage, mice were killed by decapitation and trunk blood was collected into EDTA-containing tubes. Plasma was separated by centrifugation at 3500 r.p.m. (1.4 g) for 10 min at 4 °C and stored at - 80 °C before assay. Plasma levels of anti-ClpB and anti-α-MSH IgG and IgM were assayed as described above.

### ClpB DNA assay

DNA was extracted from the cultures of the WT and ClpB mutant strains, and was also purified from mice feces using the QIAampR DNA Stool Mini Kit (Qiagen, France). Bacteria were dissolved in water and boiled at 100 °C during 5 min, after 1 min of centrifugation at 11 000 r.p.m., the supernatant containing the DNA was stored at - 20 °C. Using the NCBI primer design tool (http://www.ncbi.nlm.nih.gov/tools/primer-blast/), we designed the following nucleotide primers that amplify 180-base pair DNA region coding for the CIpB protein fragment containing one identified α-MSH-like epitope (Figure 1e), forward: 5'-GCAGCTCGAAGGCAAAACTA-3' (SEQ ID NO: 4) and reverse: 5'-ACCGCTTCGTTCTGACCAAT-3' (SED ID NO: 5) (Invitrogen Custom Primers, Cergy Pontoise, France). PCR was performed in a thermocycler with MicroAmp tubes (Eppendorf, Hambourg, Germany). The reaction was carried out in a 50-µl volume containing 25 µl of Go Taq Green Master Mix 2 × (Promega), 1 µl (20 pmol) of each primer, 21 µl of bi-distilled water and 1 µl of bacterial DNA. PCR conditions were as follows: 3 min at 94 °C followed by 35 cycles at 94 °C for 30 s, 60 °C for 30 s and 72 °C for 1.5 min. PCR products were visualized on a 1% agarose gel (Sigma), with the expected size of 180 base pair and the specificity validated using ClpB mutant strain.

### Plasma concentrations of bacterial ClpB protein

Plasma concentrations of bacterial ClpB were measured using enzyme-linked immunosorbent assay (ELISA) according to the following protocol. Rabbit polyclonal anti-CIpB IgG, customly generated by Delphi Genetics (Gosselies, Belgium), were coated on to 96-well Maxisorp plates (Nunc, Rochester, NY) using 100 µl and a concentration of 2 µg/ml in 100 mM NaHCO3 buffer, pH 9.6 for 24 h at 4°C. Plates were washed (5 min × 3) in phosphate-buffered saline (PBS) with 0.05% Tween 200, pH 7.4. The recombinant ClpB protein, customly generated by Delphi Genetics, as a standard, was serially diluted to 5, 10, 25, 50, 70, 100 and 150 pM in the sample buffer (PBS, sodium azide 0.02%, pH 7.4) and added to the wells in duplicates. The plasma samples from patients with eating disorders and healthy controls (1:25 in sample buffer) were added to the remaining wells in duplicates and the CIpB standards and plasma samples were incubated 2h at room temperature (RT). Plates were washed (5 min × 3) in PBS with 0.05% Tween 200, pH 7.4. Mouse monoclonal anti-ClpB IgG (1:500 in sample buffer), customly generated by Delphi Genetics and pre-screened for having no cross-reactivity with α-MSH, were added to the wells and incubated 90 min at room temperature. Plates were washed (5 min × 3) in PBS with 0.05% Tween 200, pH 7.4. Goat anti-mouse IgG conjugated with alkaline phosphatase (1 :2000 in sample buffer) from Jackson ImmunoResearch Laboratories, Inc. (West Grove, PA) were added to the wells and incubated for 90 min at RT. Plates were washed (5 min × 3) in PBS with 0.05% Tween 200, pH 7.4 and then 100 µl of p-nitrophenyl phosphate solution (Sigma, St. Louis, MO) was added as alkaline phosphatase substrate. After 40 min of incubation at room temperature, the reaction was stopped by adding 3N NaOH. The optical density (OD) was determined at 405 nm using a microplate reader Metertech 960 (Metertech Inc., Taipei, Taiwan). Blank OD values resulting from the reading of plates without addition of plasma samples or ClpB protein standard dilutions were subtracted from the sample OD values. Plasma concentrations of ClpB was calculated based on the OD of the CIpB standard curve and was adjusted for the plasma dilution.

### Statistical analysis

Data were analyzed and the graphs were plotted using the GraphPad Prism 5.02 (GraphPad Software Inc., San Diego, CA, USA). Normality was evaluated by the Kolmogorov-Smirnov test. Group differences were analyzed by the analysis of variance or the nonparametric Kruskal-Wallis test with the Tukey's or Dunn's post tests, respectively, according to the normality results. Body weight changes were analyzed with two-way repeated measurements analysis of variance and the Bonferroni post tests. Individual groups were compared using the Student's t-test or the Mann-Whitney test according to the normality results. Effects of absorptions of CIpB antibodies with α-MSH were analyzed using paired t-test. Pearson's or Spearman's correlation coefficients were calculated according to the normality of the variable. The cAMP production was analyzed using a nonlinear regression fit (log(α-MSH) vs normalized cAMP response), which equation was Y = 100/(1+10(LogEC50-X) × HillSlope). Data are shown as mean ± s.e.m., and for all test, Po0.05 was considered statistically significant.

### Results

### Proteomic identification of bacterial α-MSH mimetics

To identify bacterial proteins with molecular mimicry to α-MSH, a research strategy based on proteomic technology was developed. Total protein was extracted from E. coli K12 cultures, the cytoplasmic fraction was resolved by 2D gel electrophoresis (Figure 1a) and transferred to a polyvinylidene difluoride membrane. To increase the probability of detection of multiple α-MSH-like epitopes in bacterial proteins, the membrane was revealed with polyclonal anti-α-MSH IgG. 13 immunopositive protein spots were found (Figure 1b), among which the spots 1-8 disappeared after preadsorption of antibodies with 10⁻⁶M α-MSH (Figure 1c), confirming specific α-MSH-mimetic epitopes. Using mass spectrometry, protein spots 1,2,3 and 4, displaying the strongest α-MSH-like staining, were identified as isoforms of the heat-shock protein named CIpB, a 857-a.a. protein, 857 amino acid protein disaggregation chaperone or CIpB, MW 95526 (molecular weight: 95526 Da, accession number: NP_417083.1, SEQ ID NO: 1). Less intensely stained α-MSH-like spots 5-8 (with the highest MASCOT scores of 880, 877, 874 and 800, respectively) were isoforms of the 548-a.a. protein chaperonin GroEL, (molecular weight: 57293 Da; accession number: YP_001732912.1). An alternative strategy of E. coli protein separation was also used, using an OFFGEL fractionator followed by one-dimensional gel electrophoresis and western blot with anti-α-MSH IgG preadsorbed or not with α-MSH (data not shown). One band was specifically recognized by anti-α-MSH IgG and was found to contain the ClpB protein (with the highest MASCOT score of 1065). Based on these results, ClpB was selected as a target protein for further validation of its molecular mimicry with α-MSH. To analyze the amino-acid sequence homology between α-MSH and bacterial CIpB, both sequences were aligned in the Emboss Stretcher program that uses the Needleman-Wunsch algorithm (http://www.ebi.ac.uk/Tools/emboss/). The alignments revealed a site of the ClpB protein displaying discontinuous 5 a.a. sequence homology with α-MSH (Figure 1d). This putative α-MSH-like epitope was located in an inter-helical loop of the ClpB protein structure indicating that it is exposed on the protein surface, that is, accessible to auto-Abs binding. Western blot of the recombinant ClpB protein revealed with anti-α-MSH IgG showed a 96-kDa band (Figure 1e), confirming that the CIpB protein contains α-MSH-like epitope(s). These results show that the presence of at least five consecutive amino-acid sequence homology, according to the molecular mimicry concept is not an obligatory condition for bacterial proteins to be recognized by IgG crossreacting with a neuropeptide.

### Immunization of mice with ClpB

To investigate whether E. coli ClpB may induce auto-Abs crossreactive with α-MSH, influencing feeding and anxiety, mice were immunized with the recombinant bacterial ClpB protein. Mice that received CIpB together with adjuvant or adjuvant alone displayed lower body weight for a few days after injections (Figure 2a). However, 4 weeks later, CIpB-immunized mice had higher body weight (+5%) vs controls (Figure 2a). The mean daily food intake, as measured during the last 10 days of the experiment, was also higher (+13%) in ClpB-immunized mice as compared with other groups (Figure 2b). The increase in food intake was owing to increased meal size (Figure 2c), as meal number did not change (Figure 2d), indicating that the CIpB immunization interfered with satiation rather than with hunger mechanisms. This is in agreement with the known role of α-MSH to induce satiation. To further validate the relevance of ClpB immunization to α-MSH anorexigenic effect, mice received i.p. injection of α-MSH. The following 24 h food intake and body weight were not affected in ClpB-immunized mice (Figure 2e), indicating that they were not sensitive to the anorexigenic effect of administered α-MSH that was present in nonimmunized mice. After the feeding experiments, locomotor activity and anxiety related behavior in mice were studied in the open field and O-maze tests. The total locomotor activity and the time spent in the open vs border areas did not significantly differ between the study groups (data not shown). However, in the closed arms of the O-maze, the CIpB-immunized mice moved a shorter distance as compared with controls (data not shown) and spent less time as compared with all other groups (data not shown), indicating decreased anxiety. To confirm the efficiency of immunization, plasma levels of anti-CIpB IgG were assayed and their affinity measured. In CIpB immunized mice, a strong increase in anti-CIpB IgG levels (Figure 2f) with lower affinities (Figure 2g) were found, in agreement with recent IgG induction. Increased plasma levels of α-MSH-reactive IgG were also found in ClpB-immunized mice (Figure 2h); these IgG were similarly characterized by lower affinities for α-MSH, as compared with controls (Figure 2i). Adsorption of mouse plasma with α-MSH, significantly reduced plasma levels of anti-CIpB IgG, confirming that a fraction, but not all of the anti-CIpB IgG, were crossreactive with α-MSH (Figure 2f). Plasma levels of α-MSH IgM auto-Abs did not significantly differ between the groups (data not shown). Whether CIpB immunization may induce auto-Abs crossreacting with the adrenocorticotropic hormone, a 39-a.a. peptide containing the α-MSH sequence was also analyzed. No significant differences in plasma adrenocorticotropic hormone-reactive IgG were found (data not shown), showing the selectivity of the conformational mimicry between ClpB and α-MSH.

### Mouse IgG effects on MC4R signaling

To determine the impact of CIpB immunization-induced α-MSH crossreactive IgG on MC4R signaling, their effects on α-MSH-induced cAMP production in MC4R-expressing cells were studied. cAMP concentrations were found to be lower when α-MSH was preincubated with IgG from ClpB-immunized mice, as compared with α-MSH alone or α-MSH preincubated with IgG from adjuvant injected mice, with a reduction of 8-10% at the two highest α-MSH concentrations (Figure 2j). After depletion of α-MSH-reactive IgG from the pooled IgG, the remaining IgG from the ClpB immunized mice did not show any effect on α-MSH-induced cAMP release (Figure 2k), indicating that anti-α-MSH crossreactive IgG in ClpB-immunized mice were responsible for lowering cAMP production in response to α-MSH. The reduction in MC4R activation and signaling may, hence, account for the increased food intake and decreased anxiety observed in ClpB-immunized mice.

### Intragastric delivery of E. coli in mice

To test whether E. coli may induce immunogenic response against the ClpB protein, resulting in production of anti-ClpB auto-Abs crossreactive with α-MSH, WT and ΔClpB strains of E. coli K12 were given daily to mice by intragastric gavage during 3 weeks. Another group of mice was gavaged with the bacterial culture medium only, and the control group did not receive any treatment. The first days of gavage were accompanied by a decrease in body weight and food intake in mice receiving WT E.coli, which then gradually returned to control levels (Figures 3a and b). Again, during the last week of gavage, feeding pattern was affected in mice receiving E. coli WT showing a decrease in meal size but increase in meal number (Figures 3c and d). Remarkably, mice receiving ΔClpB E. coli did not significantly differ from controls in either body weight gain, food intake or feeding pattern at any time point. These data support specific involvement of bacterial ClpB in the host acute decrease of food intake as well as in the chronic regulation of feeding pattern following E. coli infection. Expectedly, CIpB DNA was more abundant in feces of mice receiving E. coli WT, although its low level was detected in some control mice (Figure 3e). After 3 weeks of gavage, plasma levels of both anti-CIpB IgM and IgG were elevated in mice that received E. coli WT as compared with controls and ΔClpB E. coli groups (Figures 3f and g). Adsorption of plasma with α-MSH reduced anti-CIpB IgG levels in E. coli WT-gavaged mice (Figure 3g), indicating the presence of anti-ClpB IgG crossreactive with α-MSH. Interestingly, plasma levels of the IgM class of anti-ClpB auto-Abs were increased after adsorption with α-MSH, suggesting that α-MSH caused dissociation of α-MSH IgM immune complexes crossreactive with ClpB that were increased in E. coli WT-gavaged mice (Figure 3f). Plasma levels of anti-α-MSH IgM were also increased by E. coli WT delivery as compared with all other groups (Figure 3h), while anti-α-MSHreactive IgG were only slightly increased without reaching significance (Figure 3i). Nevertheless, affinity kinetic analysis of α-MSH IgG revealed lower values of the dissociation equilibrium constants in E. coli-gavaged mice (Figure 3j), without significant changes of the association or dissociation rates (data not shown). These changes, including increased levels of the IgM class of α-MSH reactive auto-Abs, might reflect an immune response towards CIpB as to a novel antigen. In fact, low or undetectable levels of CIpB DNA in feces of mice that did not receive E. coli WT indicates that CIpB-expressing microorganisms were not major gut commensals in the studied mice. Thus, in contrast to ClpB-immunized mice, which showed increased levels of low-affinity anti-α-MSH IgG associated with increased meal size and body weight gain, E. coli-gavaged mice showed increased production of both anti-α-MSH-reactive IgM and IgG with increased affinities associated with decreased meal size and body weight.

### Anti-ClpB antibodies in ED patients

As the ability of the E. coli ClpB protein to stimulate production of α-MSH crossreactive auto-Abs was validated, the relevance of bacterial ClpB to ED was next determined by studying anti-ClpB antibodies in patients with AN, BN or BED. It was found that both anti-CIpB IgG and IgM were readily detectable in plasma of ED patients as well as healthy subjects with no significant differences of their mean levels (Figures 4a and b). However, there was high variability in all study groups, indicating a different individual history in encountering ClpB-like antigens. To verify whether human anti-CIpB antibodies similarly were crossreactive with α-MSH, plasma samples were adsorbed with 10⁻⁶M α-MSH, leading to significant reduction of anti-ClpB IgG and IgM detectable levels in all study groups (Figures 4c and d). Further, the relative levels of α-MSH crossreactive anti-CIpB IgG were increased in all three groups of ED patients, in particular BN and BED vs healthy controls (Figure 4e). Elevated levels of α-MSH crossreactive anti-CIpB IgM were found in AN as compared with BN (Figure 4f). To further determine the relevance of anti-CIpB IgG and IgM to ED, it was studied whether their plasma levels may correlate with behavioral traits in ED patients and controls measured by the EDI-2. It was found that in controls, CIpB IgG correlated inversely with the normal range of a few psychological traits, but in AN patients,

ClpB IgG levels correlated positively with the core psychopathological traits such as body dissatisfaction and drive for thinness (Table 1). Moreover, in AN and BED patients, EDI-2 subscale scores correlated with CIpB IgM in the opposite way, being negative in AN but positive in BED (Table 1). However, in BED patients, CIpB IgM correlated negatively with age, suggesting that the highest anti-CIpB IgM levels were associated with the acute form of the disease. Remarkably, the correlations found in AN patients between CIpB IgG or IgM and drive for thinness or interpersonal distrust, respectively, resembled closely the correlations between the same psychological traits and α-MSH-reactive IgG or IgM found in a different group of AN patients in a previous study.

**Table 1. Significant correlations between plasma levels of anti-CIpB IgG and IgM and psychological traits in eating disorder patients and controls (Contr.) assayed by the Eating Disorder Inventory-2. All Spearman's r *p<0.05, **p<0.01, except Pearson's r*p<0.05 for perfectionism. (n=65 Contr., n=27 AN, and n=14 BED).**

| | | | | |
|---|---|---|---|---|
| CIpB IgG (Contr.) | Maturity fears r= -0.31 * | Impulse regulation r= -0.26 * | Social insecurity r= -0.26 * | |
| ClpB IgG (AN) | Body dissatisfaction r= 0.4 * | Drive for thinness r= 0.35 * | Perfectionism r= 0.38 * | |
| ClpB IgM (AN) | Ineffectiveness r= -0.42 * | Interpersonal distrust r= -0.58 ** | Social insecurity r= -0.52 ** | Anhedonia r= -0.35 * |
| CIpB IgM (BED) | Bulimia r= 0.53 * | Perfectionism r= 0.6 * | Age r= -0.74 ** | |

### Plasma concentrations of bacterial ClpB protein

ClpB protein was detected in plasma samples of all study subjects ranging from 10 pM to 180 pM with a mean level of about 30 pM in healthy controls. Mean plasma levels of CIpB were significantly elevated in all groups of patients with eating disorders, including AN, BN and BED (see Figure 5). By applying the common criteria of a diagnostically-relevant changes of concentrations equal or exceeding 2 standard deviations, 21.7% of AN, 32% of BN and 25% of BED patients showed increased levels of plasma ClpB.

### Conclusion

The results reveal a molecular link between ClpB expressing gut bacteria and the regulation of motivated behavior and emotion via production of ClpB protein and anti-ClpB antibodies crossreactive with α-MSH. It shows that specific alterations of gut microbiota may lead to behavioral and emotional abnormalities as observed in ED patients. The findings of increased levels of CIpB protein and anti-ClpB IgG crossreactive with α-MSH in ED patients and correlations of anti-CIpB antibodies with patient's psychopathological traits support the involvement of CIpB-expressing microorganisms in increased CIpB antibody production and establishment of abnormal feeding behavior and emotion.

In conclusion, the results identify ClpB as a protein responsible for the origin of auto-Abs crossreactive with α-MSH, associated with psychopathological traits in ED patients and, hence, that CIpB-expressing microorganisms as a novel specific target for diagnostics and treatment of ED.

## Claims

1. An oral composition comprising probiotics overexpressing ClpB protein for use in the treatment of obesity, said ClpB protein comprising or consisting of an amino acid sequence 80 to 100% identical to the amino acid sequence of SEQ ID NO: 1.

2. The oral composition for use according to claim **1**, wherein the probiotics are commensal non-pathogenic bacteria in humans.

3. The oral composition for use according to claim **1** or claim **2**, wherein the probiotics are commensal non-pathogenic Escherichia coli.

4. The oral composition for use according to claim **1** or claim **2**, wherein the probiotics are bacteria overexpressing CIpB protein are prepared by bacterial transformation with vectors expressing CIpB DNA.

5. The oral composition for use according to any one of claims **1** to **4**, said composition being a pharmaceutical composition.

## Patentansprüche

1. Orale Zusammensetzung, die Probiotika umfasst, die ClpB-Protein überexprimieren, zur Verwendung in der Behandlung von Fettleibigkeit, wobei das ClpB-Protein eine Aminosäuresequenz, die zu 80 bis 100 % mit der Aminosäuresequenz aus SEQ. ID-Nr. 1 identisch ist, umfasst oder daraus besteht.

2. Orale Zusammensetzung zur Verwendung nach Anspruch **1**, wobei die Probiotika kommensale, bei Menschen nicht-pathogene Bakterien sind.

3. Orale Zusammensetzung zur Verwendung nach Anspruch **1** oder Anspruch **2**, wobei die Probiotika kommensale, nicht-pathogene Escherichia coli sind.

4. Orale Zusammensetzung zur Verwendung nach Anspruch **1** oder Anspruch **2**, wobei die Probiotika Bakterien sind, die ClpB-Protein überexprimieren, die durch bakterielle Transformation mit Vektoren, die ClpB-DNA exprimieren, hergestellt werden.

5. Orale Zusammensetzung zur Verwendung nach einem der Ansprüche **1** bis **4**, wobei die Zusammensetzung eine pharmazeutische Zusammensetzung ist.

## Revendications

1. Composition orale comprenant des probiotiques surexprimant la protéine ClpB pour son utilisation dans le traitement de l'obésité, ladite protéine ClpB comprenant ou consistant en une séquence d'acides aminés 80% à 100 % identique à la séquence d'acides aminés SEQ ID NO:1.

2. Composition orale pour son utilisation selon la revendication 1, **caractérisée en ce que** les prébiotiques sont des bactéries commensales non pathogènes chez l'homme.

3. Composition orale pour son utilisation selon la revendication 1 ou la revendication 2, **caractérisée en ce que** les prébiotiques sont des bactéries commensales non pathogènes Escherichia coli.

4. Composition orale pour son utilisation selon la revendication 1 ou la revendication 2, **caractérisée en ce que** les prébiotiques sont des bactéries surexprimant la protéine ClpB préparées par transformation bactérienne avec des vecteurs exprimant l'ADN ClpB.

5. Composition orale pour son utilisation selon l'une quelconque des revendications 1 à 4, ladite composition étant une composition pharmaceutique.
